Europäisches Patentamt

European Patent Office

Office européen des brevets

Publication number: **0 274 818**
**A2**

# EUROPEAN PATENT APPLICATION

Application number: 87308706.8

Date of filing: 01.10.87

Int. Cl.⁴ **G01N 33/52** , G01N 33/72 , C12Q 1/00

Priority: 16.01.87 US 3871

Date of publication of application:
**20.07.88 Bulletin 88/29**

Designated Contracting States:
**DE FR GB IT**

Applicant: **HELENA LABORATORIES**
**CORPORATION**
**1530 Lindbergh Drive**
**Beaumont Texas 77704(US)**

Inventor: **Golias, Joseph H.**
**1050 Howell**
**Beaumont Texas 77706(US)**

Representative: **Gore, Peter Manson et al**
**W.P. THOMPSON & CO. Coopers Building**
**Church Street**
**Liverpool L1 3AB(GB)**

Strip for diagnostic testing.

A device for use in a chromogen-oxygen donor test system, which comprises an elongated, continuous strip (10); said strip containing a plurality of defined test sites (18); a plurality of control sites (20) on said strip; each control site intermediate two adjacent test sites.

A device for use in determining the presence of occult blood by using a chromogen and a reagent which comprises an elongated, continuous strip (10); said strip including a plurality of test sites (18); said strip further including a plurality of control sites (20), each control site positioned intermediate to adjacent test sites; each control site for verifying the proper functioning of the chromogen and reagent utilized in the occult blood test.

Fig - 2

EP 0 274 818 A2

## STRIP FOR DIAGNOSTIC TESTING.

The present invention relates to products for diagnostic testing, such as, for example, fecal occult blood testing and, more particularly, to an improved tape or elongated continuous strip to be utilized in such testing. Because of the particular utility of the tape of the present invention in fecal occult blood testing, the tape will be explained in that context. However, the explanation should not be taken as a limitation of the utilization of the tape of the present invention.

The chemistry of fecal occult blood testing is generally understood as are the benefits of such testing, for example, to aid in early detection of gastrointestinal bleeding which may be indicative of gastrointestinal cancer.

By way of background, the test for detecting occult blood in fecal specimens is a well-known, standardized test utilizing a colourless chromogen, such as, for example guaiac, and an oxygen donor, also referred to as a developing solution, such as, for example, hydrogen peroxide. In the presence of a substance exhibiting catalytic activity such as, for example, haemoglobin, oxygen is released from the oxygen donor and reacts with the chromogen to cause a colour change in the chromogen. The test as just described is often referred to as the "guaiac" test for fecal occult blood. Usually the guaiac is printed or impregnated on a filter paper which is marketed either in the form of a slide or as a continuous strip or tape. The fecal specimen is placed on the filter paper using a spatula-type implement. Typically, when guaiac is utilized, it is printed or impregnated on conventional, absorbent filter paper such as, for example 2043A filter paper manufactured by Schleicher & Schuell. This type of filter paper has been heretofore used in slide-type products as well as tape products.

In US-A-4 541 987 other chromogens and oxygen donors are described, as well as another form of the test known as a throw-in-the-bowl where the product as marketed is dropped into a toilet bowl containing the fecal specimen.

As may be appreciated, it is desirable to verify that the chemicals, i.e., the chromogen and oxygen donor, are functioning properly. To this end, controls or monitors have heretofore been provided on or with test slides and on throw-in-the-bowl products. Exemplary prior art specifications are US-A-4 175 923, US-A-4 365 970 and US-A-4 541 987.

In addition to test slides and products to be utilized in the toilet bowl, several companies have marketed a product conventionally known as a "tape" which is, in reality, an elongated narrow thin continuous strip of material, in the form of a roll mounted in a dispenser, where short portions of the tape may be removed from a dispenser and utilized for fecal occult blood testing. The tape is most frequently utilized in hospitals and in physician's offices. However, as may be appreciated, built-in, identifiable controls or monitors have not been provided on the tape because, inter alia, different users withdraw different lengths of tape from the dispenser and, therefore, the placement of controls or monitors on the tape would interfere with the specimen test areas.

The present invention overcomes the problem referred to above by providing a new and improved tape or continuous strip test material.

More specifically, the present invention provides a continuous narrow, thin, elongated strip of test material having defined control sites or control areas to verify the proper functioning of the chemicals utilized in the fecal occult blood test and also having defined specimen test sites or test areas.

Thus the present invention overcomes the disadvantages by incorporating a control or monitor system into a tape for fecal occult blood testing or the like.

According to the present invention there is provided a device for use in a chromogen-oxygen donor test system which comprises an elongated, continuous strip; said strip containing a plurality of defined test sites; a plurality of control sites on said strip; each control site being intermediate two adjacent test sites.

The present invention also provides a device for use in determining the presence of occult blood by using a chromogen and a reagent which comprises an elongated, continuous strip; said strip including a plurality of test sites; said strip further including a plurality of control sites, each control site positioned intermediate to adjacent test sites; each control site for verifying the proper functioning of the chromogen and reagent utilized in the occult blood test.

The continuous strip may be an absorbent substrate carrying, preferably impregnated with, the chromogen.

A portion of the control site may include a substance which will give a visual indication of the proper functioning of the chromogen and reagent. Each control site may include a positive test indicator and a negative test indicator. The control site may be framed in a brightly coloured, inert border.

The strip may be provided with a plurality of means to facilitate severing the tape with each test site and one adjacent control site being positioned between two adjacent means to facilitate severing of the tape. Preferably the tape severing means is a series of perforations.

The preferred chromogen is guaiac as has previously been used in other fecal occult blood test tape and slide products. The oxygen donor recommended for use with the present invention is hydrogen peroxide as also has conventionally been used heretofore with fecal occult blood test tape and slide products.

The catalyst is a substance which is intended to cause the chromogen to undergo a colour change as long as both the chromogen and the developing solution (or reagent) have retained their desired chemical activity.

The preferred catalyst for use in the present invention is a lead acetate as described more fully in the copending, commonly owned application Serial No. 563.903 filed December 21. 1983.

The present invention will now be more particularly described wth reference to and as illustrated in, the accompanying drawings, in which:-

Figure 1 is a perspective illustration of a continuous strip or tape, in the form of a roll, mounted in a dispenser: and

Figure 2 is an illustration of a portion of the tape or continuous strip.

In the drawings, like reference numerals identify corresponding components.

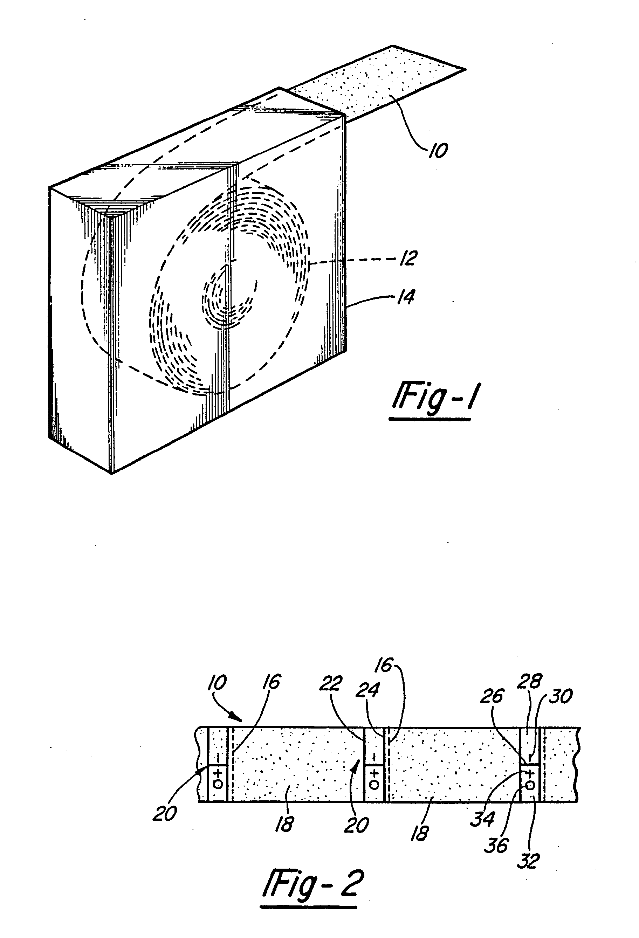

Referring to Figure 1, an elongated narrow thin continuous strip or tape 10 is illustrated wound about itself in the form of a roll 12 and positioned within a dispenser 14. The dispenser may be in the form of a rectangular solid with a suitable aperture for removal of the tape.

Referring to Figure 2, a section of the tape 10 is illustrated. One form of tape which may be utilized in the present invention is Schleicher & Schuell 2043A filter paper which is cut as an elongated, narrow, thin continuous strip. A single roll of tape may be, for example 2.06 cms (13/16 inches) wide and 254-508 cms (100-200 inches) long. The entire tape may be impregnated with the chromogen, in for example, guaiac.

The strip or tape 10 is provided with a series of spaced apart, transverse sets of perforations 16. Each set of perforations is generally perpendicular to the longitudinal axis of the strip. Each set of perforations is longitudinally spaced apart 4.44 cms (1.75 inches) from the next or adjacent set of perforations. The tape between two adjacent sets of perforations defines a specimen test site or area 18 and a control site or area 20. The specimen test area 18 may constitute the majority of the longitudinal distance between adjacent sets of perforations. for example, a distance of about 3.81 cms (about 1.50 inches) along the longitudinal axis of the strip.

The control site 20 includes a pair of spaced apart transverse lines 22,24 interconnected by a short line 26 perpendicular to lines 22,24 and thus parallel to the longitudinal axis of the tape. Line 26 may be on the central axis of the tape, thus lines 22,24 and 26 generally define the letter "H". Within one portion of the control site 20 (identified as the negative monitor area 28 and defined by lines 22,24 and 26 on one half of the longitudinal axis of the tape) a minus sign 30 is provided. On the other half of the tape 10 (defined by the lines 22,24 and 26 and being on the opposite half of longitudinal axis of the tape) is a positive monitor area 32 where a plus sign 34 is provided. For ease in discerning the lines 22,24 and 26 and the plus and minus signs, they may be printed in a bright colour such as a red or an orange utilizing a conventional ink. Printing may take place using a conventional flexographic printing technique.

The entire tape including the specimen test area 18 and control area 20 is impregnated with the chromogen. In addition, catalyst 36 is provided in the positive test area 32.

To use the tape of the present invention, the fecal specimen may be placed on the test site 18 (or more preferably on the reverse side of the tape) and then the developing solution is placed on the test site 18, in the negative monitor area 28, and in the positive monitor area 32 preferably on the catalyst 36.

A colour change in the test site 18 is considered a positive response; the absence of such a colour change is considered a negative response. In the negative part of the control site, the chromogen should remain colourless; if there is a colour change any result of the test in the test site 18 (positive or negative) should be disregarded. In the positive part of the control site, the catalyst 36 should undergo a colour change. If either the chromogen, or the oxygen donor, or both are "stale" or have lost their activity, then the catalyst remains colourless upon application of the developing solution to the catalyst, in which case any result (positive or negative) in the test site 18 should be disregarded.

As previously indicated, the tape 10 contains a plurality of sets of spaced apart perforations 16. Upon withdrawal of the tape 10 from the dispenser 14, the perforations facilitate severing a usable portion of tape which includes both a specimen test area and a control area. The perforations thus facilitate convenient separation of the tape 10 into usable sections. Obviously withdrawing a longer portion of tape 10 with several test sites and several control sites is acceptable as long as test sites and control sites are withdrawn and severed from the roll 12. The perforations 16 may be score lines or other means to facilitate severing the tape.

**Claims**

1. A device for use in a chromogen-oxygen donor test system, which comprises an elongated, continuous strip; said strip containing a plurality of defined test sites; a plurality of control sites on said strip; each control site intermediate two adjacent test sites.

2. A device for use in determining the presence of occult blood by using a chromogen and a reagent which comprises an elongated, continuous strip; said strip including a plurality of test sites; said strip further including a plurality of control sites, each control site positioned intermediate to adjacent test sites; each control site for verifying the proper functioning of the chromogen and reagent utilized in the occult blood test.

3. A device according to claim 1 or 2, wherein said continuous strip is an absorbent substrate carrying said chromogen.

4. A device according to claim 3, wherein said continuous strip is an absorbent substrate impregnated with said chromogen.

5. A device according to any of claims 2 to 4, wherein a portion of said control site includes a substance which will give a visual indication of the proper functioning of the chromogen and reagent.

6. A device according to any of claims 1 to 5, wherein each control site includes a positive test indicator and a negative test indicator.

7. A device according to any of claims 1 to 6, wherein the control site is framed by a brightly coloured, inert border.

8. A device according to any of claims 1 to 7, wherein said continuous strip is provided with a plurality of means to facilitate severing the tape; each test site and one adjacent control site being positioned between two adjacent means to facilitate severing the tape.

9. A device according to claim 8, wherein the tape severing means is a series of perforations.

10. A device according to any of claims 1 to 9, wherein the chromogen is guaiac.

11. A device according to any of claims 1 to 10, wherein the reagent is hydrogen peroxide.

12. A device according to any of claims 1 to 11, wherein the control site includes a catalyst.

13. A device according to claim 12, wherein the catalyst is lead acetate.

*IFig-1*

*IFig-2*